# EUROPEAN PATENT APPLICATION

(11) **EP 4 134 429 A1**
(43) Date of publication of application: **15.02.2023**
(21) Application number: 21784758.1
(22) Date of filing: 06.04.2021
(51) Int. Cl.: C12N 5/0775

(54) **STEM CELL CULTURING METHOD FOR PROMOTING INITIAL YIELD OF STEM CEL.LS**

(30) Priority: 08.04.2020 KR 20200042893
(71) Applicant: Encell Co., Ltd., Gangnam-gu, Seoul 06072 (KR); Samsung Life Public Welfare Foundation, Seoul 04348 (KR)
(72) Inventor: CHANG, Jong Wook, Seoul 06351 (KR); JEON, Hong Bae, Seoul 05531 (KR); PARK, Sang Eon, Seongnam-si, Gyeonggi-do 13500 (KR)
(74) Representative: dompatent von Kreisler Selting Werner - Partnerschaft von Patent- und Rechtsanwälten mbB
(86) International application number: PCT/KR2021/004244
(87) International publication number: WO 2021/206402

(57) **Abstract**

The present invention provides a method of culturing mesenchymal stem cells, comprising performing primary culture of umbilical cord-derived mesenchymal stem cells under hypoxic conditions with an oxygen partial pressure of 1 to 8% and pressure conditions of 1.0 to 8.0 PSI. The method of culturing umbilical cord-derived mesenchymal stem cells according to the present invention can maintain stemness by inhibiting the differentiation of stem cells and particularly can achieve a remarkably high proliferation rate.

## Description

### [Technical Field]

This application claims the benefit of Korean Patent Application No. 10-2020-0042893 filed on April 8, 2020, with the Korean Intellectual Property Office, the disclosure of which is herein incorporated by reference in its entirety.

The present invention relates to a method of culturing stem cells, and to a method of promoting the initial yield of stem cell production.

### [Background Art]

Stem cells can be differentiated into various cells, and various studies are being conducted on the applicability of cell therapy using the characteristics of these stem cells. As a result, the number of papers, patents and clinical trials of stem cell therapeutic agents is on the rise, and the global market size for stem cell therapeutic agents is projected to be more than $100 billion as of 2018 (Transparency Market Research, 2019).

Embryonic stem cells with pluripotency have been attracting attention as cell therapeutic agents because of their ability to differentiate into various cells, but have difficulties in practical application due to safety and ethical issues.

In order to avoid these safety and ethical issues of embryonic stem cells, researchers have become interested in adult stem cells that can replace embryonic stem cells, and are actively conducting studies using mesenchymal stem cells, which have high self-replication ability and can differentiate into various tissues, among them.

Mesenchymal stem cells can be regenerated into muscle, cartilage, bone tissue, ligaments, bone marrow stroma, and the like through differentiation, and various proteins secreted by themselves have therapeutic effects.

Specifically, through paracrine action of various proteins secreted by mesenchymal stem cells, the action of inducing homeostasis recovery of an individual such as inhibition of inflammation and/or immune regulation at the lesion site is known. Because of this paracrine action, mesenchymal stem cells are called "drug store" or "drug factory."

As a mesenchymal stem cell therapeutic agent, seven therapeutic agents are currently on the market worldwide, and four of them were first approved and marketed in Korea. For the development of cell therapeutic agents using adult stem cells without ethical issues, it is essential to establish a culturing method that may effectively proliferate the cells while maintaining the stemness of the cells.

However, since adult stem cells have a low proliferation rate, there is a problem in that the number of cells that can be obtained from one tissue is limited. In general, it is known that bone marrow-derived adult stem cells have very limited ability to differentiate into various tissues, and it is not easy to obtain many cells. Other types of adult stem cells, i.e., umbilical cord blood or adipose stem cells, are relatively easy to obtain, but have limited differentiation capacity like bone marrow-derived adult stem cells.

In general, in order for stem cell therapeutic agents to be clinically applied, the number of cells above a certain level (at least 1×10⁹ cells) is required, and for this, mass culture of stem cells is essential. However, in the process of culturing stem cells, aging of stem cells inevitably occurs as the culture period becomes longer, which leads to a decrease in stemness such as proliferative capacity or differentiation capacity and therapeutic efficacy. In addition, since there are disadvantages in that the production cost such as material cost and labor cost increases as the culture period becomes longer, the need for a culturing method for proliferating the mesenchymal stem cells while maintaining the stemness is increasing.

Compared to other adult stem cells, umbilical cord-derived stem cells, which have recently been attracting attention from researchers, do not require invasive procedures and have low immunogenicity, and thus, they have the advantage of being able to transplant not only autologous but also allogenic stem cells. However, since the number of cells that can be isolated from one umbilical cord is limited, when cell culture is performed to increase the number of cells, the cells age and the time during which the cell proliferation is maintained is reduced, and thus, there is the same problem that the number of cells that can be obtained is small.

Therefore, for the development of cell therapeutic agents using adult stem cells, i.e., mesenchymal stem cells, it is required to develop a method capable of effectively proliferating the cells while maintaining stemness.

### [Prior Art Documents]

Korean Patent No. 1624514
Korean Patent Publication No. 2008-0026415

### [Disclosure]

### [Technical Problem]

The present invention has been devised to solve the problem of the conventional low stem cell production yield, and it is an object of the present invention to provide a stem cell culturing method for increasing the initial yield of stem cells and maintaining the stemness of stem cells.

### [Technical Solution]

In order to achieve the above object, the present invention provides a method of culturing stem cells, comprising performing primary culture of the stem cells under hypoxic conditions with an oxygen partial pressure of 1 to 8% and pressure conditions of 1.0 to 8.0 pound per square inch (PSI).

### [Advantageous Effects]

According to the method of culturing stem cells of the present invention, the initial yield of stem cells is increased by about 3 times compared to the conventional culturing method, and a high stem cell proliferation rate can be achieved by the doubling time reduced by about 10 hours on average compared to the conventional culturing method.

In addition, the method of culturing stem cells of the present invention provides an effect of maintaining stemness by inhibiting the differentiation of stem cells.

In addition, the method of culturing stem cells of the present invention enables mass production of stem cells by shortening the culture rate of stem cells.

### [Description of Drawings]

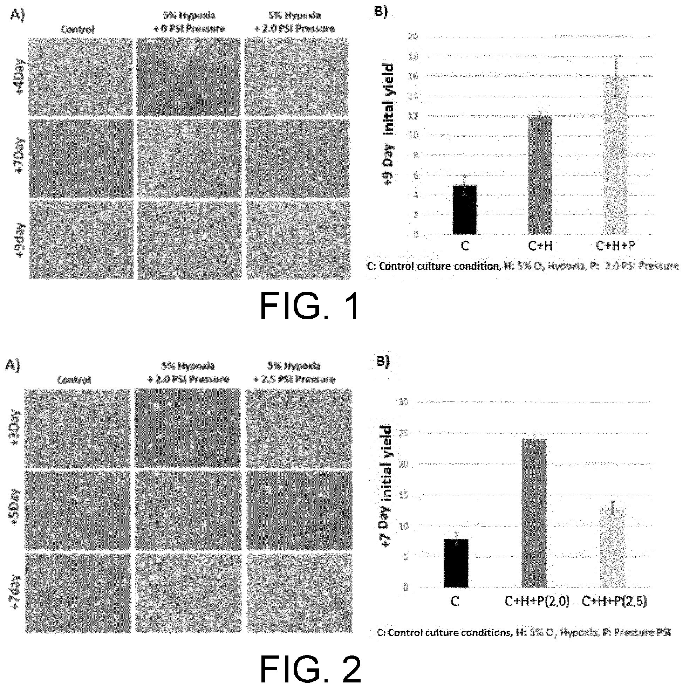

Figure 1 shows the results of performing primary culture of the human umbilical cord tissue-derived mesenchymal stem cells obtained by the method of Example 1 under normoxic conditions (C), hypoxic conditions (H), or hypoxic conditions and pressure conditions (H+P). Figure 1A shows the results of observing the shape of stem cells with an optical microscope after culture for 4 days, 7 days, and 9 days under each condition, and Figure 1B shows the cell yield when primary culture is performed for 9 days under each condition.
Figure 2 shows the results of performing primary culture of the human umbilical cord tissue-derived mesenchymal stem cells obtained by the method of Example 1 under normoxic conditions (C), hypoxic conditions (H) and pressure conditions of 2.0 PSI (C+H+P(2.0)), or hypoxic conditions and pressure conditions of 2.5 PSI (C+H+P (2.5)) . Figure 2A shows the results observed with an optical microscope on the 3rd, 5th, and 7th days under each condition, respectively, and Figure 2B shows the cell yield when primary culture is performed for 7 days under each condition.

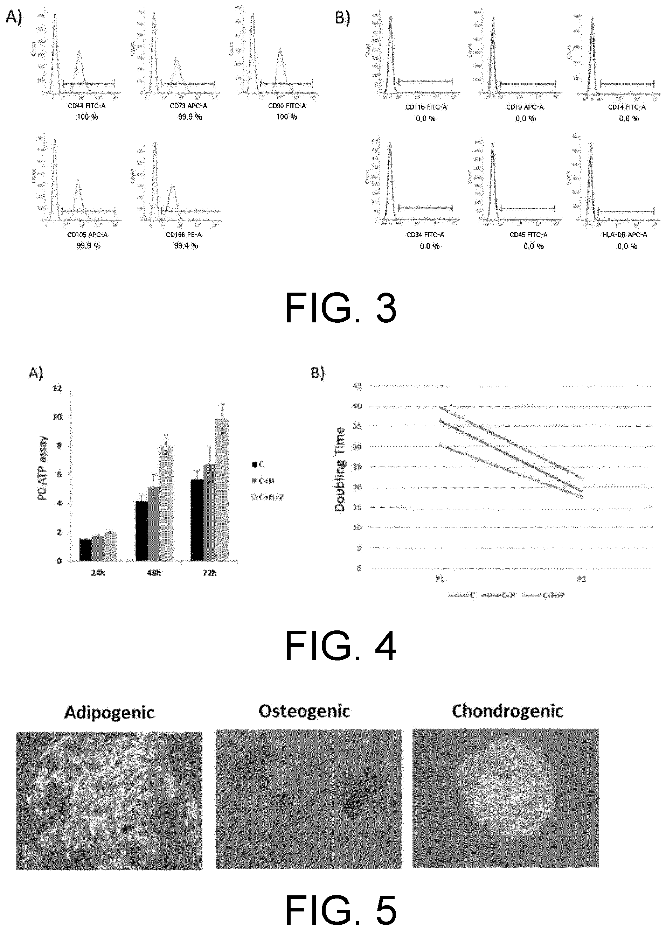

Figure 3 shows the expression level of the mesenchymal stem cell markers (Figure 3A) and the negative markers (Figure 3B) after the primary culture of the human umbilical cord tissue-derived mesenchymal stem cells obtained by the method of Example 1.
Figure 4 shows the results of performing primary culture or subculture of the human umbilical cord tissue-derived mesenchymal stem cells obtained by the method of Example 1 under normoxic conditions (C), hypoxic conditions (H), or hypoxic conditions and pressure conditions (C+H+P). Figure 4A shows the amount of ATP produced after the primary culture for 24 hours, 48 hours, and 72 hours under the same conditions, and Figure 4B shows the doubling time during P1 and P2 culture.
Figure 5 shows a staining result specific for each differentiated cell after the primary culture of the human umbilical cord tissue-derived mesenchymal stem cells obtained by the method of Example 1 under hypoxic conditions and pressure conditions of 2.0 PSI, followed by each differentiation using a differentiation medium for differentiating into adipocytes, osteoblasts, or chondrocytes.

### [Best Mode]

Hereinafter, the present invention will be described in more detail.

The present invention relates to a method of culturing stem cells, comprising performing primary culture of the stem cells under hypoxic conditions with an oxygen partial pressure of 1 to 8% and pressure conditions of 1.0 to 8.0 pound per square inch (PSI), and mesenchymal stem cells cultured by the method.

The mesenchymal stem cells determined by the International Society for Cellular Therapy (ISCT) should grow attached to the bottom when cultured, be able to differentiate into osteoblasts, adipocytes or chondrocytes *in vitro,* and express CD73, CD90, CD105, CD166 and CD44 as cell surface markers, but should not express CD34, CD45, CD19, CD11b, CD14 and HLA-DR.

The method of culturing stem cells of the present invention may be applied to mesenchymal stem cells derived from various origin tissues. Examples of the mesenchymal stem cells may include, but are not limited to, mesenchymal stem cells derived from umbilical cord blood, placenta, umbilical cord, bone marrow, adipose tissue, and the like. In one embodiment of the present invention, the method of culturing stem cells of the present invention is applied to umbilical cord-derived mesenchymal stem cells.

In addition, the method of culturing mesenchymal stem cells of the present invention may be applied to mesenchymal stem cells obtained from various subjects. Examples of the mesenchymal stem cells may include, but are not limited to, mesenchymal stem cells obtained from mammals including humans. In one embodiment of the present invention, the method of culturing mesenchymal stem cells of the present invention is applied to mesenchymal stem cells obtained from humans, and in another embodiment, it is applied to human umbilical cord mesenchymal stem cells.

Adult stem cells have completely different factors affecting stemness maintenance and proliferation depending on the source from which they are derived. For example, if adipose tissue-derived stem cells and bone marrow-derived stem cells are cultured under hypoxic conditions, differentiation of bone marrow-derived stem cells is induced depending on the type of stem cells (J Cell Physiol. 2006 May;207(2):331-9), and differentiation of adipose tissue-derived stem cells is inhibited (J Biol Chem. 2006 Oct 13;281(41):30678-83. Epub 2006 Aug 22).

In addition, it has been reported that umbilical cord blood-derived mesenchymal stem cells are differentiated into cardiomyocytes when a periodic pressure change is applied for culture in a medium containing BMP-2 protein (Korean Patent Application Laid-Open No. 10-2008-0026415), and it has been reported that bone marrow-derived mesenchymal stem cells induce bone/cartilage differentiation when pressure is applied (Stem Cell Research(2015) 14, 283-296).

The culturing method of the present invention is characterized by performing primary culture of the stem cells under hypoxic conditions. The hypoxic conditions further promote the proliferation of stem cells compared to the normoxia conditions, which mean the oxygen concentration in the atmosphere. In order to achieve the above object, oxygen may be used in a concentration of 1 to 8%, in a concentration of 2 to 8%, in a concentration of 3 to 8%, in a concentration of 4 to 8%, in a concentration of 2 to 6%, in a concentration of 3 to 6%, in a concentration of 4 to 6%, in a concentration of 2 to 5%, or in a concentration of 3 to 5%.

Also, the oxygen may be used in a concentration of 1%, 2%, 3%, 4%, 5%, 6%, 7%, or 8%.

When the concentration of oxygen is less than 1% or more than 8%, there is a problem in that the proliferation of stem cells is remarkably reduced. In one embodiment of the present invention, the hypoxic conditions are an oxygen concentration of 5%.

The hypoxic conditions are achieved by controlling the oxygen concentration within the cell incubator. For example, the hypoxic conditions may be achieved by supplying nitrogen gas (100%) or nitrogen/carbon dioxide (95%/5%) mixed gas into an incubator having normoxic conditions to replace the air in the incubator. The oxygen concentration may be confirmed through an oxygen sensor mounted on the incubator.

The culturing method of the present invention is characterized by performing primary culture of the stem cells under pressure conditions. The pressure conditions further promote the proliferation of stem cells compared to atmospheric conditions. In order to achieve the above object, the pressure conditions of 1.0 to 8.0 pressure per square inch (PSI), 2.0 to 8.0 PSI, 3.0 to 8.0 PSI, 4.0 to 8.0 PSI, 5.0 to 8.0 PSI, 6.0 to 8.0 PSI, 1.0 to 6.0 PSI, 2.0 to 6.0 PSI, 3.0 to 6.0 PSI, 4.0 to 6.0 PSI, 1.0 to 4.0 PSI, 2.0 to 4.0 PSI, 3.0 to 4.0 PSI, or 1.0 to 4.0 PSI may be used.

In addition, the pressure conditions of 1 PSI, 2 PSI, 3 PSI, 4 PSI, 5 PSI, 6 PSI, 7 PSI, or 8 PSI may be used.

When the pressure is less than 1.0 PSI or more than 8.0 PSI, there is a problem in that the proliferation of stem cells is remarkably reduced. In one embodiment of the present invention, the pressure conditions are 2.0 PSI or 2.5 PSI.

In addition, the culturing method of the present invention is characterized by performing primary culture of the stem cells under hypoxic conditions with an oxygen concentration of 2 to 5% and pressure conditions of 1.0 to 8.0 PSI. One embodiment of the present invention is hypoxic conditions with an oxygen concentration of 5% and pressure conditions of 2.0 PSI. Another embodiment of the present invention is hypoxic conditions with an oxygen concentration of 5% and pressure conditions of 2.5 PSI.

In addition to the above-mentioned conditions, the culture of mesenchymal stem cells is performed by a method of regulating oxygen concentration and pressure through the exchange of air in the cell culture device with the air in the pressure change device using the inhalation and exhalation valves in a conventionally used cell incubator. An example of the cell incubator may include, but is not limited to, an Avatar^{™} Cell Control System (xcellbio) capable of simultaneously controlling the oxygen concentration and pressure in the incubator.

The "primary culture" is to isolate cells from tissue and culture them for the first time. Since the mesenchymal stem cells are adherent culture cells, the nutrient medium may be changed if necessary, but the culture vessel is not changed. In the present invention, mesenchymal stem cells isolated from the umbilical cord are first cultured in an incubator.

The "initial yield" is the number of cells proliferated by the primary culture. In the present invention, it is the number of cells when performing primary culture of mesenchymal stem cells isolated from the umbilical cord in which the primary culture was performed.

The "subculture" is to proliferate and maintain the previously cultured cells after transfer to a new culture vessel. Since the mesenchymal stem cells isolated from the umbilical cord, which are the cells of the present invention, are adherent culture cells, they are removed from the culture vessel by treatment with enzymes such as trypsin, and then transferred to a new culture vessel and subcultured.

The culturing method of the present invention may be applied during primary culture or subculture of stem cells. Since the proliferation of stem cells is promoted when primary culture of the stem cells is performed in the method of the present invention, there is an advantage in that a large number of stem cells may be obtained even with a small number of subcultures.

In addition, since the mesenchymal stem cells obtained through the culturing method of the present invention do not have immunogenicity and do not induce an immune response, they may be effectively used as cell therapeutic agents for humans. Accordingly, the present invention provides mesenchymal stem cells with improved proliferative power, viability, recovery rate, and the like, obtained by the culturing method. In addition, the present invention provides a cell therapeutic agent comprising the mesenchymal stem cells obtained by the culturing method. The cell therapeutic agent of the present invention may be used for regeneration or protection of adipocytes, osteocytes, chondrocytes, muscle cells, nerve cells, cardiomyocytes, hepatocytes, pancreatic islet beta-cells, vascular cells, lung cells, or the like.

Hereinafter, preferred examples will be provided to help understanding the present invention, but the following examples are only for illustrating the present invention. It will be apparent to those skilled in the art that various changes and modifications may be made within the scope and technical spirit of the present invention, and it is obvious that such changes and modifications fall within the scope of the appended claims.

### [Mode for Invention]

### Example 1: Isolation of umbilical cord tissue-derived mesenchymal stem cells

Human umbilical cord tissue was collected with the consent of the mothers after obtaining approval from the Institutional Review Board (IRB) of Samsung Medical Center.

The collected umbilical cord was treated according to some modifications to the previously reported method of Peng J et al. (Brain Research Bulletin, 84 (2011) 235-234), wherein the umbilical cord was washed several times with phosphate buffered saline (PBS), and then cut to a length of 3 to 4 cm, and crushed finely using scissors after removing blood vessels and amniotic membrane. The crushed tissue was treated with 2 mg/ml of collagenase at a temperature of 37°C for 60 minutes to isolate cells. After the enzyme reaction was stopped by treating the isolated cell fraction with fetal bovine serum, the enzyme reaction solution was centrifuged at 1,000 g at room temperature for 10 minutes to obtain cells.

After the obtained cells were washed with serum-free a-modified minimum essential media (aMEM), the cells were added to aMEM containing 15% fetal bovine serum and 0.5% gentamicin (10 mg/ml) and counted to 50,000 to 100,000 cells per cm², and primary culture (P0) was performed.

The first medium change was performed after 4 days of primary culture, and when the cell confluence reached 70-80%, the attached cells were floated using 0.25% trypsin. After the cells obtained by floating were washed with serum-free aMEM, the cells were added to aMEM containing 10% fetal bovine serum and 0.5 % gentamicin (10 mg/ml) and counted to 3,000 to 5,000 cells per cm², and subculture (P1) was performed.

As a result of analyzing the surface markers of the cells obtained by subculturing up to P2, the expression of markers (CD90, CD105, CD73, CD166, and CD44) of mesenchymal stem cells was shown, but the negative markers (CD34, CD45, CD19, CD11b, CD14, and HLA-DR) of mesenchymal stem cells were not expressed (not shown).

### Example 2: Ex vivo culture of umbilical cord tissue-derived mesenchymal stem cells

An experiment was performed to analyze the initial yield according to the culture conditions during primary culture (P0) of the umbilical cord tissue-derived mesenchymal stem cells isolated in Example 1.

In a specific experimental method, primary culture (P0) of the umbilical cord tissue-derived mesenchymal stem cells isolated in Example 1 was performed under normoxic conditions (Control), hypoxic conditions (5% O₂ Hypoxia), and pressure conditions (pressure of 2.0 PSI) alone or in combination in the same medium used in Example 1.

The results observed with an optical microscope on the 4th, 7th and 9th days from the start of the culture, respectively, are as shown in Figure 1A. From the results of Figure 1A, it could be confirmed that all cells cultured under normoxic conditions (C), hypoxic conditions (C+H), and hypoxic conditions + pressure conditions (C+H+P) were cultured while maintaining the shape of mesenchymal stem cells.

In addition, the cell yield (%) by 9-day culture under the same conditions was confirmed by the formula of "(number of cells obtained through primary culture/number of cells isolated from umbilical cord tissue) X 100," and the results are as shown in Figure 1B.

From the results of Figure 1B, it can be confirmed that the initial yield of 5% (normoxic conditions; C) in the 9-day culture of the umbilical cord tissue-derived mesenchymal stem cells obtained by the method of Example 1 increases by 12.5% under hypoxic conditions alone (C+H), and 16% under the combination of hypoxic conditions and pressure conditions (C+H+P).

### Example 3: Ex vivo culture of umbilical cord tissue-derived mesenchymal stem cells

An experiment was performed to optimize the pressure conditions during primary culture (P0) of the umbilical cord tissue-derived mesenchymal stem cells isolated in Example 1.

In a specific experimental method, primary culture of the umbilical cord tissue-derived mesenchymal stem cells isolated in Example 1 was performed under normoxic conditions (Control), hypoxic conditions and pressure conditions of 2.0 PSI (5% O₂ Hypoxia + 2.0 PSI), or hypoxic conditions and pressure conditions of 2.5 PSI (5% O₂ Hypoxia + 2.5 PSI) in the same medium used in Example 1.

The results observed with an optical microscope on the 3rd, 5th and 7th days from the start of the culture, respectively, are as shown in Figure 2A. From the results of Figure 2A, it could be confirmed that all cells cultured under normoxic conditions (Control), and hypoxic conditions and pressure conditions (5% O₂ Hypoxia + 2.0 PSI, 5% O₂ Hypoxia + 2.5 PSI) were cultured while maintaining the shape of mesenchymal stem cells.

In addition, the cell yield by 7-day culture under the same conditions was confirmed in the same manner as in Example 2, and the results are as shown in Figure 2B. From the results of Figure 2B, it can be confirmed that compared to the initial yield of 8% (normoxic conditions; C) in the 7-day culture of the umbilical cord tissue-derived mesenchymal stem cells obtained by the method of Example 1, the initial yield in the culture under hypoxia and 2.0 PSI (5% Hypoxia + 2.0 PSI; C+H+P(2)) increases by 24%, and the initial yield in the culture under hypoxia and 2.5 PSI (5% Hypoxia + 2.5 PSI; C+H+P(2.5)) increases by 13%.

Therefore, the optimum pressure condition for the primary culture of umbilical cord tissue-derived mesenchymal stem cells was selected as 2.0 PSI.

### Example 4: Analysis of cell surface markers after primary culture and subculture of umbilical cord tissue-derived mesenchymal stem cells

Analysis of cell surface markers was performed to confirm whether the properties of the mesenchymal stem cells were maintained after the primary culture of the umbilical cord tissue-derived mesenchymal stem cells isolated in Example 1.

In a specific experimental method, cells obtained by performing primary culture of the umbilical cord tissue-derived mesenchymal stem cells isolated in Example 1 for 7 days under hypoxic conditions and pressure conditions (5% hypoxia + 2.0 PSI) in the same medium used in Example 1 were subcultured under normoxic and pressure conditions up to P2 to obtain cells. The obtained cells were analyzed for expression patterns of mesenchymal stem cell-specific cell surface markers (CD90, CD105, CD73, CD166, and CD44) according to the requirements of the International Society for Cellular Therapy (ISCT), and cells expressing negative markers (CD34, CD45, CD19, CD11b, CD14, and HLA-DR) of mesenchymal stem cells were analyzed for purity analysis by flow cytometry.

As a result, it could be confirmed that the umbilical cord tissue-derived mesenchymal stem cells subcultured after primary culture under hypoxic conditions and pressure conditions still expressed 99% or more of mesenchymal stem cell-specific cell surface markers (Figure 3A), but did not express negative markers (Figure 3B).

### Example 5: Analysis of proliferative capacity during primary culture of umbilical cord tissue-derived mesenchymal stem cells

An experiment was performed to analyze the proliferative capacity according to the culture conditions during primary culture (P0) of the umbilical cord tissue-derived mesenchymal stem cells isolated in Example 1.

In a specific experimental method, primary culture (P0) of the umbilical cord tissue-derived mesenchymal stem cells isolated in Example 1 was performed under normoxic conditions (C), hypoxic conditions (5% O₂ Hypoxia; C+H), and hypoxic conditions and pressure conditions (pressure of 2.0 PSI; C+H+P) in the same medium used in Example 1.

Cells were collected at 24 hours, 48 hours, and 72 hours after the start of the culture, respectively, and the amount of intracellular ATP produced was analyzed. ATP analysis was performed with Promega Corporation's CellTiter-Glo^{®} kit according to the instructions, and the results are as shown in Figure 4A.

From the results of Figure 4A, it can be confirmed that in the umbilical cord tissue-derived mesenchymal stem cells, the amount of ATP produced under hypoxic conditions and pressure conditions (C+H+P) remarkably increases at each time period compared to normoxic conditions (C) and hypoxic conditions (C+H).

In addition, an experiment was performed to analyze the doubling time according to the culture conditions during subculture of the umbilical cord tissue-derived mesenchymal stem cells isolated in Example 1.

In a specific experimental method, primary culture (P0) of the umbilical cord tissue-derived mesenchymal stem cells isolated in Example 1 was performed under normoxic conditions (C), hypoxic conditions (5% O₂ Hypoxia; C+H), or hypoxic conditions and pressure conditions (pressure of 2.0 PSI; C+H+P) in the same medium used in Example 1, and subcultures (P1, P2) were performed under normoxic and pressure conditions.

From the results of Figure 4B, it could be confirmed that in the umbilical cord tissue-derived mesenchymal stem cells cultured under hypoxic conditions (C+H), or hypoxic conditions and pressure conditions (C+H+P) after the primary culture (P0), the doubling time was shortened by 3 hours and 9 hours, respectively, compared to the existing conditions even during P1 and P2 cultures under normoxic and pressure conditions.

### Example 6: Measurement of differentiation capacity after ex vivo culture of umbilical cord tissue-derived mesenchymal stem cells

After the primary culture of the umbilical cord tissue-derived mesenchymal stem cells isolated in Example 1, they were differentiated using a differentiation medium for differentiating into typical mesenchymal cells such as adipocytes, osteoblasts, and chondrocytes.

In a specific experimental method, cells obtained by performing primary culture (PO) of the umbilical cord tissue-derived mesenchymal stem cells isolated in Example 1 for 7 days under hypoxic conditions (5% O₂) and pressure conditions of 2.0 PSI in the same medium used in Example 1 were subcultured under normoxic and pressure conditions up to P2 to obtain cells. It was confirmed whether the obtained cells were differentiated into individual cells after the culture for 10 to 30 days in a differentiation medium for differentiating into osteoblasts, chondrocytes, or adipocytes.

StemPro Adipogenesis Differentiation Kit was used for adipocyte differentiation, StemPro Osteogenesis Differentiation Kit was used for osteoblast differentiation, and a medium containing BMP-6, TGFβ3, insulin-transferrin-selenium (ITS), dexamethasone, ascorbic acid, L-proline, and sodium pyruvate in DMEM medium was used for chondrocyte differentiation.

Adipocytes, osteoblasts, and chondrocytes were stained with oil-red O, Alizarin red S, and Safranin-O, respectively, and the results are as shown in Figure 5.

From the results of Figure 5, it could be confirmed that the umbilical cord tissue-derived mesenchymal stem cells, in which the primary culture was performed under hypoxic conditions and pressure conditions, were differentiated into mesenchymal cells such as adipocytes, osteoblasts, or chondrocytes in a suitable differentiation medium.

Although the present invention has been described above with reference to limited examples and drawings, the present invention is not limited thereto, and it will be apparent that various modifications and variations may be made within the scope of the technical spirit of the present invention and equivalents of the claims to be described below by those skilled in the art to which the present invention pertains.

## Claims

1. A method of culturing stem cells, comprising performing primary culture of the stem cells under hypoxic conditions with an oxygen partial pressure of 1 to 8% and pressure conditions of 1.0 to 8.0 pound per square inch (PSI).

2. The method of culturing stem cells according to claim 1, wherein the oxygen partial pressure is 5%.

3. The method of culturing stem cells according to claim 1, wherein the pressure condition is 2.0 PSI.

4. The method of culturing stem cells according to claim 1, wherein the pressure condition is 2.5 PSI.

5. The method of culturing stem cells according to claim 1, wherein the stem cells are mesenchymal stem cells derived from bone marrow stroma, adipose tissue, cartilage, or umbilical cord.

6. The method of culturing stem cells according to claim 5, wherein the mesenchymal stem cells are derived from the umbilical cord.

7. The method of culturing stem cells according to any one of claims 1 to 6, wherein the culture is primary culture for 5 to 15 days.

8. The method of culturing stem cells according to claim 7, wherein the culture is primary culture for 4 to 9 days.

9. The method of culturing stem cells according to claim 8, wherein the culture is performed in a-modified minimum essential media (aMEM) comprising fetal bovine serum and antibiotics.

10. The method of culturing stem cells according to claim 9, wherein the culture is performed in a-modified minimum essential media (aMEM) comprising 15% fetal bovine serum and 0.5% gentamicin.
